(19) Europäisches Patentamt / European Patent Office / Office européen des brevets

(11) **EP 1 132 069 B1**

(12) **EUROPEAN PATENT SPECIFICATION**

(45) Date of publication and mention
of the grant of the patent:
**06.01.2010 Bulletin 2010/01**

(51) Int Cl.:
***A61F 13/475*** (2006.01)   ***A61F 13/494*** (2006.01)

(21) Application number: **01302019.3**

(22) Date of filing: **06.03.2001**

(54) **ABSORBENT ARTICLE**

ABSORBIERENDER ARTIKEL

ARTICLE ABSORBANT

(84) Designated Contracting States:
**DE FR GB NL SE**

(30) Priority: **06.03.2000   JP 2000060246**

(43) Date of publication of application:
**12.09.2001   Bulletin 2001/37**

(73) Proprietor: **UNI-CHARM CORPORATION**
**Shikokuchuo-shi,**
**Ehime-ken (JP)**

(72) Inventors:
• **Mizutani, Satoshi,**
**c/o Technical Center**
**Kanonji-shi**
**Kagawa-ken 769-1602, Japan (JP)**
• **Suekane, Makoto,**
**c/o Technical Center**
**Kanonji-shi,**
**Kagawa-ken 769-1602, Japan (JP)**

• **Nishitani, Kazuya,**
**c/o Technical Center**
**Kanonji-shi,**
**Kagawa-ken 769-1602, Japan (JP)**

(74) Representative: **Eke, Philippa Dianne et al**
**Saunders & Dolleymore LLP**
**9 Rickmansworth Road**
**Watford**
**Hertfordshire WD18 0JU (GB)**

(56) References cited:
| | |
|---|---|
| EP-A- 0 876 810 | EP-A- 0 904 759 |
| EP-A- 0 962 208 | WO-A-95/11649 |
| WO-A-97/09016 | WO-A-97/09017 |
| GB-A- 2 319 730 | US-A- 5 167 653 |
| US-A- 5 312 386 | US-A- 5 456 971 |
| US-A- 5 807 363 | |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND OF THE INVENTION

1. Field of the Invention

The present invention relates to an absorbent article such as a sanitary napkin or a pantie liner and, more particularly, to an absorbent article which prevents leakage of a liquid waste.

2. Related Art

The sanitary napkin has to prevent the so-called "sideway leaking phenomenon", in which a liquid waste such as menstrual blood migrates so far transversely of the worn napkin as to leak out from the side edge portions of the napkin. In the sanitary napkin of this kind of the prior art, therefore, there have been known various counter-measures for preventing the sideway leakage.

The sideway leakage of the liquid waste is prevented, for example, by providing a sanitary napkin with leakage preventing walls. These leakage preventing walls are disposed on widthwise two sides of a surface sheet of the sanitary napkin, which comes into direct contact with the private part (e.g., the vaginal opening) of a wearer, and extend in the longitudinal direction so that the liquid waste given to the surface sheet hardly flows over the leakage preventing walls.

However, since this leakage preventing wall does not absorb the liquid waste sufficiently, the liquid waste dammed up by the leakage preventing wall is left thereon as a residual liquid. As a result, the humidity in the space between the wearer's skin and the sanitary napkin is increased thereby to leave a problem that the wearer cannot enjoy a comfortable dry or unwet feel.

It is also known to provide an absorbent sheet in such a leakage preventing wall. With this absorbent sheet, the liquid waste flowing into the leakage preventing wall can be absorbed therein thereby to improve the sideway leakage preventing effect.

However, the liquid waste absorbed by the absorbent sheet does not migrate into an absorbent core located below the surface sheet smoothly. Therefore, the liquid waste received by the leakage preventing wall is liable to be retained only in the leakage preventing wall. In this case, since the liquid absorbing capacity of the leakage preventing wall per se is not high, its allowable limit is easily exceeded. Therefore, if the liquid waste is given to the leakage preventing wall at an amount exceeding the allowable limit, the liquid waste flows back to blot the skin or underwear.

Moreover, the leakage preventing wall having absorbed the liquid waste is liable to deform due to its expansion in volume as a whole or due to the weight of the absorbed liquid waste. As a result, the leakage preventing wall has its expected shape broken to deteriorate the pliability (or the soft feel or the cushioning properties). As the case may be, the leakage preventing walls are folded or stepped to cause the wearer to feel pains by the pressure or a physical disorder.

SUMMARY OF THE INVENTION

The invention has an object to provide an absorbent article which can prevent the sideway leakage of liquid waste.

The present invention provides the absorbent article of independent claim 1. The dependent claims specify preferred but optional features.

According to an aspect of the invention, there is provided an absorbent article comprising a main body including: a liquid-permeable surface sheet; a back sheet; and an absorbent core sandwiched between the surface sheet and the back sheet, and two leakage preventing walls extending in the longitudinal direction of the main body and provided on two sides of the main body lying opposite one another in the widthwise direction thereof,

the overlap of the absorbent core and the surface sheet including: a main absorbent portion located between the two leakage preventing walls and at the center of the overlap in the widthwise direction; and two grooved portions formed by depressing the overlap and each located between the main absorbent portion and corresponding one of the two leakage preventing walls,
wherein the fiber density of the portion of the surface sheet forming the main absorbent portion, the fiber density of the portion of the surface sheet forming the grooved portion and the fiber density of the leakage preventing wall are related in the order of the grooved portion > the leakage preventing wall > the main absorbent portion.

According to the invention, due to its density gradient, the liquid waste in the grooved portion can be prevented from flowing back to the main absorbent region and the leakage preventing wall, so that the liquid waste can be diffused into the grooved portion and further smoothly transferred through the grooved portion into the absorbent core.

Preferably, each leakage preventing wall is formed by folding a sheet to have at least two free ends.

Preferably, the leakage preventing wall is formed of a hydrophilic sheet or a hydrophobic sheet.

Preferably, the absorbent article has two flaps extending outwardly of the absorbent core in the widthwise direction. In this case, it is also preferred that two side sheets are provided on flap-forming portions of the back sheet and are individually folded at least one time to form the leakage preventing walls.

Preferably, the leakage preventing wall has a height of 3 mm to 20 mm.

Preferably, an elastically stretchable member is disposed in the leakage preventing wall and extends in the longitudinal direction.

In order to make the fiber density of the portion of the surface sheet forming the grooved portion higher than those of the others, it is preferred that the absorbent core and the surface sheet are thermally compressed at the grooved portion.

In an alternative, it is preferred that the portion of the surface sheet forming the main absorbent portion, the portion of the surface sheet forming the grooved portion, and the leakage preventing wall are made of different sheet materials, and that if the fineness of fibers forming the portion of the surface sheet forming the main absorbent portion, the fineness of fibers forming the leakage preventing wall, and the fineness of fibers forming the portion of the surface sheet forming the grooved portion are designated by D1, D2 and D3, respectively, the individual finenesses are D1 = 2 to 6 dtex, D2 = 1.5 to 6 dtex, and D3 = 1 to 4.5 dtex, and have relations of D1 < D2 < D3.

In another alternative, it is preferred that fusible fibers contained in the surface sheet and the leakage preventing wall are thermo-compression bonded, and that if the bonding area percentages in the portion of the surface sheet forming the main absorbent portion, in the leakage preventing wall, and in the portion of the surface sheet forming the grooved portion are designated by P1, P2 and P3, respectively, the individual percentages are within the ranges of P1 = 0 to 60 %, P2 = 0 to 70 %, and P3 = 0 to 80 %, and have relations of P1 < P2 < P3.

BRIEF DESCRIPTION OF THE DRAWINGS

Fig. 1 is a top plan view showing a sanitary napkin as an absorbent article according to a first embodiment of the invention;

Fig. 2 is a sectional view showing a portion on the Y1 side, as taken along line II - II of Fig. 1;

Fig. 3 is a sectional view showing a portion on the right side (on the X1 side), as taken along line III - III of Fig. 1; and

Fig. 4 is a sectional view showing a portion on the right side (on the X1 side) of a sanitary napkin according to a second embodiment.

DESCRIPTION OF THE PREFERRED EMBODIMENTS

The invention will be described with reference to the accompanying drawings.

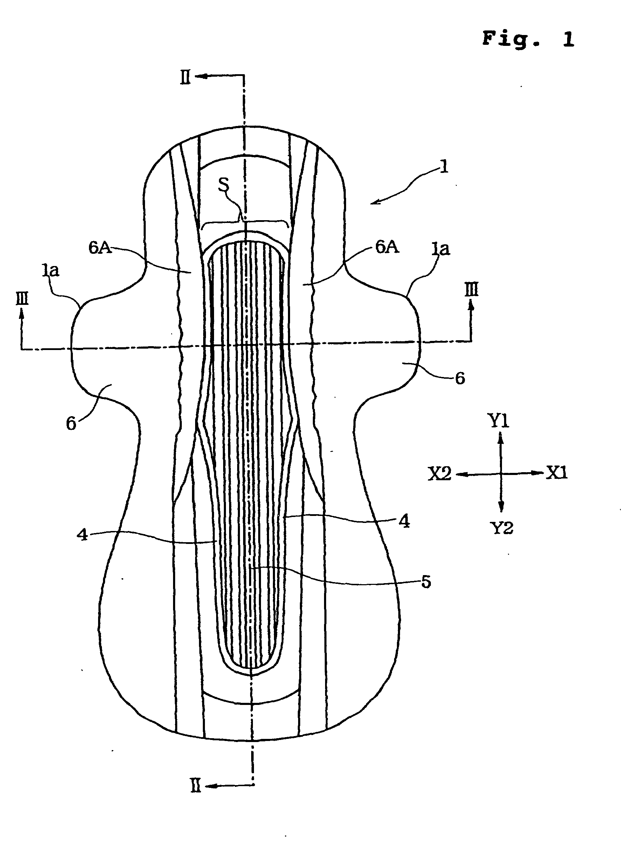

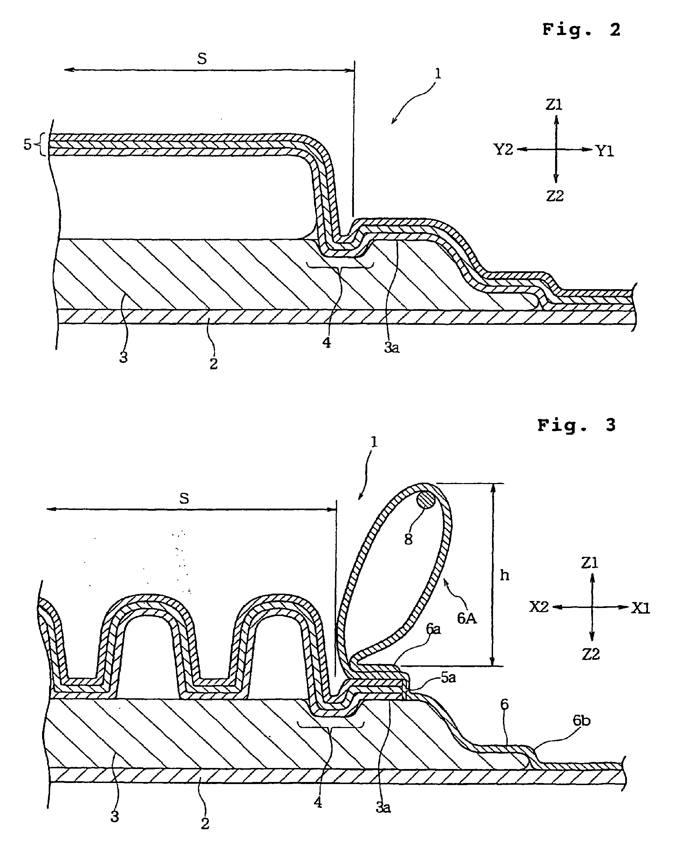

Fig. 1 is a top plan view showing a sanitary napkin as an absorbent article according to a first embodiment of the invention; Fig. 2 is a sectional view showing a portion on the Y1 side, as taken along line II - II of Fig. 1; and Fig. 3 is a sectional view showing a portion on the right side (on the X1 side), as taken along line III - III of Fig. 1.

A sanitary napkin (or an absorbent article) 1, as shown in Figs. 1 to 3, is provided at its lowermost layer with a back sheet 2 which is formed of a liquid-impermeable sheet. The back sheet 2 is formed with flaps 1a and 1a on two sides lying opposite one another in widthwise direction (i.e., in the direction X1 - X2). Between these flaps 1a and 1a, there is disposed an absorbent core 3 on the back sheet 2.

Over the absorbent core 3, there is disposed a surface sheet 5 which is formed of a liquid-permeable fibrous sheet. In the embodiment shown, the surface sheet 5 is of a sectionally corrugated shape which is composed of ridge portions and valley portions. These back sheet2, surface sheet 5 and absorbent core 3 form a main body of the sanitary napkin 1.

In the absorbent article 1, there is formed a continuous groove (or channel) including right and left grooved portions 4 and 4, as shown in Figs. 1 to 3, in which the fibrous sheet forming the surface sheet 5 is thermally compressed and attached (or joined) to the absorbent core 3. As shown in Fig. 1, the continuous groove is formed at the center of the surface sheet 5 with the right and left grooved portions 4 and 4 being continuous with each other. However, the right and left grooved portions 4 and 4 may be separated form each other. It should be noted that the thermal compression may be done either all along the grooved portion 4 or partially (e.g., intermittently) along the grooved portion 4. In either event, the fiber density of the surface sheet 5 in the grooved portion 4 is raised to give an excellent water absorbing capacity. A peripheral portion 5a of the surface sheet 5, as extending outwardly of the grooved portions 4, is attached to a peripheral portion 3a of the absorbent core 3, as extending outwardly of the grooved portions 4, by means of a hot-melt adhesive or the like.

In this absorbent article 1, the whole area, in which the absorbent core 3 and the surface sheet 5 overlap each other, is the absorbent area. Especially, in this absorbent area, the portion enclosed by the continuous groove (or sandwiched between the right and left grooved portions 4 and 4, if separated) is designated "main absorbent portion S", which is to be brought into direct contact with the vaginal opening (or the private part), from which the liquid waste such as the menstrual blood is discharged.

On two sides of the absorbent article 1, as shown in Figs. 1 and 3, there are provided side sheets 6 and 6 each extending from the peripheral portion 3a of the absorbent core 3 to the flap 1a. These side sheets 6 and 6 are formed of a hydrophilic or hydrophobic nonwoven fabric. On the inner sides (or main absorbent portion-facing sides) of the side sheets 6 and 6, there are formed leakage preventing walls 6A and 6A. Each leakage preventing wall 6A is formed by folding back the inner side portion 6a of the side sheet 6. As shown in Fig. 3, along the folds (or free ends) of the leakage preventing walls 6A, there are disposed elastically stretchable members 8. These elastically stretchable members 8 are wholly or partially fixed by a hot-melt adhesive, a heat-sealing method or the like to the inner faces of the leakage preventing walls 6A to extend in the longitudinal direction.

As indicated by solid lines in Fig. 1, the root ends of the leakage preventing walls 6A and 6A extend generally arcuately and partially along the grooved portions 4 and 4. In the sectional view of Fig. 3, the root end of the leakage preventing wall 6A is attached to the peripheral portion 3a of the absorbent core 3 through the peripheral portion 5a of the surface sheet 5. Remaining front and rear end portions (i.e., the portions not forming the leakage preventing walls 6A) of the folded inner side portions 6a of the side sheets 6, as located longitudinally outwardly of the aforementioned arcuate solid lines, are fixed substantially entirely on the main body. The outer side portions indicated at 6b of the side sheets 6 are also fixed on the main body (e.g., peripheral portion 3a of the absorbent core 3 or the back sheet 2) by a hot-melt adhesive or the like.

The leakage preventing walls 6A are hard to erect if their height h (See Fig. 3) is less than 3 mm. If the height h exceeds 20 mm, on the contrary, the wearer finds it difficult to wear the absorbent article or feels uncomfortable. Therefore, the height h of the leakage preventing walls 6A is set within a range of 3 to 20 mm.

The surface sheet 5 can be formed of a nonwoven fabric, which is formed of hydrophobic fibers subjected to a hydrophilic treatment or hydrophilic fibers and is excellent in a liquid permeability. This nonwoven fabric may be of either a single-layered structure or a multi-layered structure (e.g., a three-layered structure, as shown in Figs. 2 and 3). In the latter case, the nonwoven fabric preferably has a total basis weight (this may be referred to as "METSUKE") of about 85 $g/m^2$.

The elastically stretchable members 8 are stretchable members made of natural rubber or urethane, for example. The leakage preventing walls 6A are hard to erect, if the elastically stretchable members 8 have a length less than 30 mm in the longitudinal direction (or the direction Y), but give the physical disorder to the wearer if the length exceeds 300 mm. Therefore, the length of the members 8 is preferred to be within a range of 30 to 300 mm. If the magnification of elastic stretch of the elastically stretchable members 8 is 1, the leakage preventing walls 6A are hard to erect. If the magnification exceeds 2.5 times, the wearer is given the feel of physical disorder. Therefore, the magnification of elastic stretch is preferred to be within a range of more than 1 to 2.5 times. When the elongation percentage of the elastically stretchable members 8 is 50 % (e.g., when a member 8 of 100 mm is stretched to 150mm), the tensile load of the members 8 is preferred to be within a range of $98 \times 10^{-3}$ N to 3 N (i.e., a range of 10 gf to about 300 gf). If the tensile load is less than $98 \times 10^{-3}$ N (10 gf), the leakage preventing walls 6A are hard to erect. If the tensile load is more than 3 N (about 300 gf), the wearer feels the physical disorder. On the other hand, the distortion (or the ratio of the deformation to the original length) of the elastically stretchable members 8 is preferred to be 30 % or less, after the elastically stretchable members 8 were elongated at 50% (e.g., from 100 mm to 150 mm) at a tension speed of 100 mm/min and then released from the tension. This is because the leakage preventing walls 6A are hard to erect if the distortion exceeds 30 %.

With the elastically stretchable members 8 thus being provided inside of the leakage preventing walls 6A, the leakage preventing walls 6A can enhance the followability to the intense motions of the wearer. Then, the liquid waste around the private part can be wiped off, or the liquid waste can be dammed up for preventing the outward leakage of liquid waste. The constituent fibers of the nonwoven fabric forming the leakage preventing wall 6A (or the side sheet 6) are preferred to have a fineness of 0.1 dtex to 4.5 dtex, at least in the portion where the elastically stretchable member 8 is fixed. This is because the fiber strength is short for the fineness less than 0.1 dtex whereas the feeling is deteriorated for the fineness more than 4.5 dtex. Since this portion of the nonwoven fabric where the member 8 is fixed is always in contact with the skin of the wearer, moreover, it is preferred to be softened by opening or shaping it.

The back sheet 2 can be formed of an olefin resin sheet, a nonwoven fabric, a laminate of the resin sheet and the nonwoven fabric or the like. The absorbent core 3 can be made of pulverized pulp, a mixture of pulverized pulp and a highly water-absorbent polymer (i.e., super absorbent polymer) or the like, which is enveloped by an absorbent sheet such as tissue paper. These components can be joined to one another by a hot-melt adhesive, as described hereinbefore, but may be joined to one another by thermal fusion with ultrasonic waves or by a heating method.

In the sanitary napkin (or the absorbent article) 1, the individual surfaces to confront the private part are made to have different fiber densities. When the individual fiber densities are compared among the surfaces of the sanitary napkin 1 at the main absorbent portion S, the grooved portion 4 and the leakage preventing wall 6A, more specifically, the grooved portion 4 has the highest fiber density, followed by the leakage preventing wall 6A, and the main absorbent portion S has the lowest fiber density (that is, the grooved portion 4 > the leakage preventing wall 6A > the main absorbent portion S).

With the fiber densities of the individual portions being thus given the density gradient, the liquid waste in the grooved portion 4 can be prevented from flowing back by the capillary phenomenon and accordingly from infiltrating into the surface sheet 5 of the main absorbent portion S and/or the leakage preventing wall 6A.

On the contrary, the liquid waste, as received by the surface sheet 5 of the main absorbent portion S or the leakage preventing wall 6A, is easily infiltrated toward the grooved portion 4 having the high fiber density so that it can be diffused into the grooved portion 4 and further smoothly transferred through the grooved portion 4 into the absorbent core 3. Especially, the liquid waste is not confined in the leakage preventing wall 6A so that the sanitary napkin to be provided can be excellent in the dry or unwet feel at all times.

If the leakage preventing walls 6A (or the side sheets 6) are formed of a hydrophilic or hydrophobic nonwoven fabric or a nonwoven fabric having a little absorbing capacity, the liquid waste received by the leakage preventing walls 6A can be more effectively guided into the grooved portion 4 and the absorbent core 3.

It should be noted that the shape of the leakage preventing walls is not limited to that of the first embodiment. For example, the leakage preventing walls may be shaped to have the following shape.

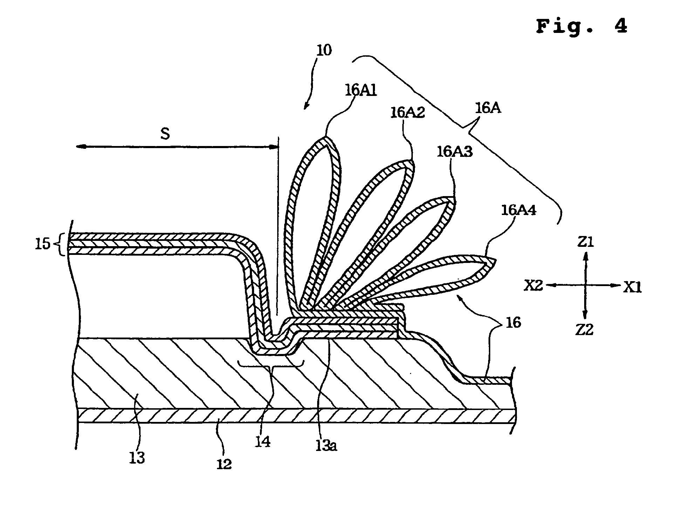

Fig. 4 is a sectional view of a sanitary napkin as an absorbent article according to a second embodiment of the invention and shows a portion on the right side (on the X1 side).

A sanitary napkin 10, as shown in Fig. 4, has a construction substantially similar to that of the sanitary napkin 1 according to the foregoing first embodiment. Over a back sheet 12, more specifically, there are laid an absorbent core 13 and a surface sheet 15, which are joined each other at a thermally compressed continuous groove (or grooved portions 14). Side sheets 16 are provided on two sides of the sanitary napkin 10.

However, the difference resides in that a plurality of (e.g., four in Fig. 4) leakage preventing walls 16A (as individually indicated at 16A1, 16A2, 16A3 and 16A4) are provided mainly on the peripheral portion 13a of the absorbent core 13. Specifically, the leakage preventing walls 16A are formed by folding back the side sheet 16 a plurality of times, and their folded portions to be root ends of the leakage preventing walls 16A are individually attached to the peripheral portion 13a of the absorbent core 13 (with the surface sheet 15 interposed therebetween).

In this embodiment, additionally, the surface sheet 15 is not corrugated, but extends generally flat at a position away from the absorbent core 3 in the main absorbent portion S.

The individual leakage preventing walls 16A1, 16A2, 16A3 and 16A4 are formed to extend in the longitudinal direction over the peripheral portion 13a, and their innermost one (as located on the side of the main absorbent portion S) is supported by the others on the outer side. Specifically: the leakage preventing walls 16A1 are supported by the leakage preventing walls 16A2; the leakage preventing walls 16A2 are supported by the leakage preventing walls 16A3; and the leakage preventing walls 16A3 are supported by the leakage preventing walls 16A4. Moreover, the leakage preventing walls 16A individually have the air layers (or hollows) formed therein so that they are given a high elastic action (or a cushioning action) partially or as a whole. As shown in Fig. 4, therefore, between two adjacent leakage preventing walls, the wall on the side closer to the main absorbent portion S (i.e., on the X2 side) can erect more easily in the height direction (i.e., in the direction Z1 - Z2). As a result, the innermost leakage preventing walls 16A1 can erect without providing the elastically stretchable member 8 therein, as different from the first embodiment. However, if the leakage preventing walls 16A are few in number, it is preferred that the elastically stretchable member 8 is disposed at least in the innermost leakage preventing wall 16A1 as in the foregoing embodiment. If necessary, the elastically stretchable members 8 may be disposed in the remaining outer leakage preventing walls 16A2, 16A3 and 16A4.

If the individual fiber densities are compared among the surfaces of the sanitary napkin 10 at the main absorbent portion S, the grooved portion 14 and the leakage preventing wall 16A, the grooved portion 14 has the highest fiber density, followed by the leakage preventing wall 16A, and the main absorbent portion S has the lowest fiber density (that is, the grooved portion 14 > the leakage preventing wall 16A > the main absorbent portion S), as in the foregoing first embodiment. As a result, the liquid waste, as received especially by the leakage preventing walls 16A, is easily infiltrated through the grooved portion 14 into the absorbent core 13. Therefore, the leakage preventing walls 16A can be made excellent in the dry or unwet feel and difficult to get out of shape.

In the foregoing embodiments, the surface sheet 5 (or 15) is thermally compressed in the grooved portions 4 (or 14) for giving the different fiber densities (or the density gradient). However, the following means can also be adopted for giving the different fiber densities.

(1) The density gradient can be set by preparing the individual fibrous sheets of fibers of different finenesses. In this case, the surface sheet is formed by joining two kinds of fibrous sheets. More specifically, it is advisable: that the fineness D1 of the constituent fibers of the fibrous sheet forming the main absorbent portion S of the surface sheet 5 is in a range between 2 and 6 dtex, the fineness D2 of the constituent fibers of the fibrous sheet (i.e., the side sheet 6) forming the leakage preventing wall 6A is in a range between 1.5 and 6 dtex, and the fineness D3 of the constituent fibers of the fibrous sheet forming the grooved portion 4 of the surface sheet 5 is in a range between 1 and 4.5 dtex; and that the individual finenesses have relations of D1 < D2 < D3. It should be noted that the lower limits of the ranges of those individual finenesses are determined to prevent an occurrence of residual liquid; the upper limits thereof are determined to prevent deterioration of the feeling.

(2) The density gradient can also be set by adjusting the percentages of bonding areas in individual portions (i.e., the portion forming the main absorbent portion S of the surface sheet 5; the portion forming the leakage preventing walls 6A; and the portion forming the grooved portion 4 of the surface sheet 5). In this case, the surface sheet 5 may be formed either of one fibrous sheet or by joining two kinds of fibrous sheets. More specifically, if the surface sheet 5 and the side sheet 6 are formed by thermo-compression bonding fusible fibers contained therein, the density gradient can be set by adjusting the percentages of thermo-compression bonding areas (i.e., (the thermo-compression bonding point area in a unit area / the unit area) x 100). If the percentage P1 of the surface sheet 5 in the main absorbent portion S is in a range between 0 and 60 %, the percentage P2 of the side sheet 6 forming the leakage preventing wall 6A is in a range between 0 and 70 %, and the percentage P3 of the surface sheet 5 in the grooved portion 4 is in a range between 0 and 80 %, and if the individual percentages are set to P1 < P2 < P3, for example, the density gradient can be set such that the fiber densities are in the order of the grooved portion > the leakage preventing wall > the main absorbent portion. If the percentages P1, P2 and P3 exceed the aforementioned upper limits, the fibers become too hard.

It should be noted that the density gradient may be set without thermally compressing the surface sheet 5 in the grooved portion 4 by adjusting the finenesses or the bonding area percentages, as described in (1) and (2), but even when the density gradient are set as in (1) and (2), the thermal compression is preferably carried out to emphasize the density gradient.

In the main absorbent portion S, the surface sheet 5 comes into direct contact with the private part (or the vicinity of the vaginal opening) of the wearer from which the liquid waste is discharged. In order that the surface sheet 5 may continue the contact with the private part without any physical disorder i.e., in order to obtain an excellent soft feel or cushioning properties, the pliability (or flexibility) of the surface sheet 5 is preferably higher than that of the private part. Therefore, the compressive forces of the individual portions are measured to determine the proper range of the pliability of the surface sheet 5, as exemplified in the following.

[Method of Measuring the Pliability]

Measured were compressive forces acting on the private part, the thigh (near the crotch) in the vicinity of the private part, the surface sheet 5 and the leakage preventing walls 6A when a cylindrical metal rod having a diameter of 5 mm is applied to depress these portions by 5mm.

[Results of the Pliability]

Compressive force of the private part

$$\risingdotseq 7 \times 10^{-3} \ (Kgf) = 68.6 \times 10^{-3} \ (N);$$

Compressive force of the thigh near the private part

$$= 13 \times 10^{-3} \ (Kgf) = 127.4 \times 10^{-3} \ (N);$$

Compressive force of the surface sheet

$$= 3 \times 10^{-3} \ (Kgf) = 29.4 \times 10^{-3} \ (N); \text{ and}$$

## Compressive force of the leakage preventing walls

$$= 10 \times 10^{-3} \text{ (Kgf)} = 98 \times 10^{-3} \text{ (N)}.$$

From these results, it is found that the compressive force of the surface sheet 5 is preferably less than 68.6 x $10^{-3}$ (N). On the other hand, since the leakage preventing walls 6A come into direct contact with the thigh near the private part, their pliability is preferably higher than that of the thigh near the private part. Therefore, it is also found that the compressive force of the leakage preventing walls 6A is preferably less than 127.4 x $10^{-3}$ (N). As understood from the results, the sanitary napkin 1 of the invention has a sufficient pliability.

According to the invention, as has been described in detail, the liquid waste received by the leakage preventing walls can be smoothly transferred to the absorbent core through the grooved portion having a high fiber density, thereby to prevent the sideway leakage of the liquid waste.

## Claims

1. An absorbent article (1) comprising a main body including: a liquid-permeable surface sheet (5); a back sheet (2); an absorbent core (3) sandwiched between said surface sheet and said back sheet, and a side sheet (6) on each of two sides of the absorbent article, wherein a leakage preventing wall (6A) is formed by folding a side sheet to have at least two free ends, the two leakage preventing walls (6A) extending in the longitudinal direction of said main body and being provided on two sides of said main body lying opposite one another in the widthwise direction thereof,
the overlap of said absorbent core and said surface sheet including: a main absorbent portion (S) located between said two leakage preventing walls and at the center of said overlap in the widthwise direction; and two grooved portions (4) formed by depressing said overlap and each located between said main absorbent portion and corresponding one of said two leakage preventing walls,
wherein the fiber density of the portion of said surface sheet forming said main absorbent portion (S), the fiber density of the portion of said surface sheet forming said grooved portion (4) and the fiber density of said leakage preventing wall (6A) are related in the order of said grooved portion > said leakage preventing wall > said main absorbent portion.

2. An absorbent article as claimed in Claim 1,
wherein said leakage preventing wall is formed of a hydrophilic sheet or a hydrophobic sheet.

3. An absorbent article as claimed in Claim 1 or Claim 2,
which has two flaps (1a) extending outwardly of said absorbent core in the widthwise direction,
wherein the two side sheets (6) are provided on flap-forming portions of said back sheet and are individually folded at least one time to form said leakage preventing walls.

4. An absorbent article as claimed in Claim 1,
wherein said leakage preventing wall has a height of 3 mm to 20 mm.

5. An absorbent article as claimed in any preceding claim,
wherein an elastically stretchable member (8) is disposed in said leakage preventing wall and extends in the longitudinal direction.

6. An absorbent article as claimed in any preceding claim,
wherein said absorbent core and said surface sheet are thermally compressed at said grooved portion.

7. An absorbent article as claimed in any preceding claim,
wherein the portion of said surface sheet forming said main absorbent portion, the portion of said surface sheet forming said grooved portion, and said leakage preventing wall are made of different sheet materials, and
wherein if the fineness of fibers forming the portion of said surface sheet forming said main absorbent portion, the fineness of fibers forming said leakage preventing wall, sand the fineness of fibers forming the portion of said surface sheet forming said grooved portion are designated by D1, D2 and D3, respectively, the individual finenesses are D1 = 2 to 6 dtex, D2 = 1.5 to 6 dtex, and D3 = 1 to 4.5 dtex, and have relations of D1 < D2 < D3.

**8.** An absorbent article as claimed in any one of Claims 1 to 6,
wherein fusible fibers contained in said surface sheet and said leakage preventing wall are thermo-compression bonded, and
wherein if the bonding area percentages in the portion of said surface sheet forming said main absorbent portion, in said leakage preventing wall, and in the portion of said surface sheet forming said grooved portion are designated by P1, P2 and P3, respectively, the individual percentages are within the ranges of P1 = 0 to 60 %, P2 = 0 to 70 %, and P3 = 0 to 80 %, and have relations of P1 < P2 < P3.


**Patentansprüche**

**1.** Absorbierender Artikel (1) mit einem Hauptteil umfassend: eine flüssigkeitsdurchlässige Oberflächenlage (5); eine rückseitige Lage (2); einen zwischen der besagten Oberflächenlage und der besagten rückseitigen Lage angeordneten absorbierenden Kern (3); und jeweils eine Seitenlage (6) auf beiden Seiten des absorbierenden Artikels, wobei durch Falten einer Seitenlage zu mindestens zwei freien Enden eine Leckschutzwand (6A) gebildet wird, die beiden Leckschutzwände (6A) sich in Längsrichtung des besagten Hauptteils erstrecken und auf zwei Seiten des besagten Hauptteils einander in Querrichtung desselben gegenüber liegen,
wobei die Überlappung des besagten absorbierenden Kerns und der besagten Oberflächenlage umfasst: einen absorbierenden Hauptabschnitt (S) zwischen den besagten beiden Leckschutzwänden und in der Mitte der besagten Überlappung in Querrichtung; und zwei durch Eindrücken der besagten Überlappung gebildete Rillenabschnitte (4), die jeweils zwischen dem besagten absorbierenden Hauptabschnitt und der entsprechenden Leckschutzwand angeordnet sind,
wobei die Faserdichte des den besagten absorbierenden Hauptabschnitt (S) bildenden Abschnitts der besagten Oberflächenlage, die Faserdichte des den besagten Rillenabschnitt (4) bildenden Abschnitts der besagten Oberflächenlage und die Faserdichte der besagten Leckschutzwand (6A) im folgenden Verhältnis zueinander stehen: besagter Rillenabschnitt > besagte Leckschutzwand > besagter absorbierender Hauptabschnitt.

**2.** Absorbierender Artikel nach Anspruch 1,
wobei die besagte Leckschutzwand aus einer hydrophilen Lage oder aus einer hydrophoben Lage gebildet ist.

**3.** Absorbierender Artikel nach Anspruch 1 oder 2,
der zwei sich außerhalb des besagten absorbierenden Kerns in Querrichtung erstreckende Klappen (1a) aufweist, wobei die beiden Seitenlagen (6) an Klappen bildenden Abschnitten der besagten rückseitigen Lage vorgesehen sind und zur Bildung der besagten Leckschutzwände mindestens einmal einzeln gefaltet werden.

**4.** Absorbierender Artikel nach Anspruch 1,
wobei die besagte Leckschutzwand eine Höhe von 3 mm bis 20 mm hat.

**5.** Absorbierender Artikel nach einem der vorhergehenden Ansprüche,
wobei in der besagten Leckschutzwand ein elastisch dehnbares Element (8) angeordnet ist und sich in Längsrichtung erstreckt.

**6.** Absorbierender Artikel nach einem der vorhergehenden Ansprüche,
wobei der besagte absorbierende Kern und die besagte Oberflächenlage im Bereich des besagten Rillenabschnitts unter Wärmeeinwirkung eingedrückt werden.

**7.** Absorbierender Artikel nach einem der vorhergehenden Ansprüche,
wobei der den besagten absorbierenden Hauptabschnitt bildende Abschnitt der besagten Oberflächenlage, der den besagten Rillenabschnitt bildende Abschnitt der besagten Oberflächenlage und die besagte Leckschutzwand aus unterschiedlichen Stoffen bestehen, und
wobei, wenn die Feinheit der Fasern des den besagten absorbierenden Hauptabschnitt bildenden Abschnitts der besagten Oberflächenlage, die Feinheit der die besagte Leckschutzwand bildenden Fasern und die Feinheit der Fasern des den besagten Rillenabschnitt bildenden Abschnitts der besagten Oberflächenlage mit D1, D2 bzw. D3 bezeichnet werden, die Feinheitsgrade D1 = 2 bis 6 dtex, D2 = 1,5 bis 6 dtex und D3 = 1 bis 4,5 dtex betragen und im Verhältnis D1 < D2 < D3 zueinander stehen.

**8.** Absorbierender Artikel nach einem der Ansprüche 1 bis 6,
wobei in der besagten Oberflächenlage und in der besagten Leckschutzwand enthaltene Schmelzfasern im Thermokompressionsverfahren verschweißt werden, und
wobei, wenn die Prozentwerte der Schweißfläche in dem den besagten absorbierenden Hauptabschnitt bildenden Abschnitt der besagten Oberflächenlage, in der besagten Leckschutzwand und in dem den besagten Rillenabschnitt bildenden Abschnitt der besagten Oberflächenlage mit P1, P2, bzw. P3 bezeichnet werden, die einzelnen Prozentwerte im Bereich P 1 = 0 bis 60%, P2 = 0 bis 70% und P3 = 0 bis 80% liegen und im Verhältnis P1 < P2 < P3 zueinander stehen.

**Revendications**

1. Article absorbant (1) comportant un corps principal comprenant : une feuille de surface perméable aux liquides (5) ; une feuille de base (2) ; une partie centrale absorbante (3) prise en sandwich entre ladite feuille de surface et ladite feuille de base, et une feuille latérale (6) sur chacun des deux côtés de l'article absorbant, dans lequel une paroi antifuite (6A) est formée en pliant une feuille latérale de sorte à avoir au moins deux extrémités libres, les deux parois antifuite (6A) s'étendant dans le sens longitudinal dudit corps principal, et étant mises en oeuvre des deux côtés dudit corps principal reposant de manière opposée l'une par rapport à l'autre dans le sens de la largeur de celui-ci,
le recouvrement de ladite partie centrale absorbante et de ladite feuille de surface comprenant : une partie absorbante principale (S) située entre lesdites deux parois antifuite et au centre dudit recouvrement dans le sens de la largeur ; et deux parties rainurées (4) formées en appuyant sur ledit recouvrement et chacune étant située entre ladite partie absorbante principale et l'une quelconque correspondante desdites parois antifuite,
dans lequel la densité des fibres de la partie de ladite feuille de surface formant ladite partie absorbante principale (S), la densité des fibres de la partie de ladite feuille de surface formant ladite partie rainurée (4) et la densité des fibres de ladite paroi antifuite (6A) sont reliées selon l'ordre suivant de ladite partie rainurée > ladite paroi antifuite > ladite partie absorbante principale.

2. Article absorbant selon la revendication 1,
dans lequel ladite paroi antifuite est formée à partir d'une feuille hydrophile ou d'une feuille hydrophobe.

3. Article absorbant selon la revendication 1 ou la revendication 2,
qui a deux rabats (1a) s'étendant vers l'extérieur de ladite partie centrale absorbante dans le sens de la largeur,
dans lequel les deux feuilles latérales (6) sont mises en oeuvre sur des parties formant rabat de ladite feuille de base et sont pliées individuellement au moins une fois pour former lesdites parois antifuite.

4. Article absorbant selon la revendication 1,
dans lequel ladite paroi antifuite a une hauteur de 3 mm à 20 mm.

5. Article absorbant selon l'une quelconque des revendications précédentes,
dans lequel un élément élastiquement extensible (8) est disposé dans ladite paroi antifuite et s'étend dans le sens longitudinal.

6. Article absorbant selon l'une quelconque des revendications précédentes,
dans lequel ladite partie centrale absorbante et ladite feuille de surface sont thermiquement comprimées au niveau de ladite partie rainurée.

7. Article absorbant selon l'une quelconque des revendications précédentes,
dans lequel la partie de ladite feuille de surface formant ladite partie absorbante principale, la partie de ladite feuille de surface formant ladite partie rainurée, et ladite paroi antifuite sont réalisées à partir de différents matériaux de feuille, et
dans lequel, si la finesse des fibres formant la partie de ladite feuille de surface formant ladite partie absorbante principale, la finesse des fibres formant ladite paroi antifuite, et la finesse des fibres formant la partie de ladite feuille de surface formant ladite partie rainurée sont désignées par D1, D2 et D3, respectivement, les finesses individuelles sont D1 = 2 à 6 dtex, D2 = 1,5 à 6 dtex, et D3 = 1 à 4,5 dtex, et ont les relations suivantes D1 < D2 < D3.

8. Article absorbant selon l'une quelconque des revendications 1 à 6,
dans lequel des fibres fusibles contenues dans ladite feuille de surface et ladite paroi antifuite sont soudées par thermo-compression, et
dans lequel, si les pourcentages de zone de soudage dans la partie de ladite feuille de surface formant ladite partie

EP 1 132 069 B1

absorbante principale, dans ladite paroi antifuite, et dans la partie de ladite feuille de surface formant ladite partie rainurée sont désignés par P1, P2 et P3, respectivement, les pourcentages individuels sont de l'ordre de P1 = 0 à 60 %, P2 = 0 à 70 %, et P3 = 0 à 80 %, et ont les relations suivantes P1 < P2 < P3.

Fig. 1

Fig. 2

Fig. 3

EP 1 132 069 B1

Fig. 4

13